# EUROPEAN PATENT SPECIFICATION

(11) **EP 2 061 529 B1**
(45) Date of publication and mention of the grant of the patent: **24.07.2013**
(21) Application number: 07842500.6
(22) Date of filing: 14.09.2007
(51) Int. Cl.: A61L 31/10, A61L 31/14

(54) **MEDICAL COMPONENTS HAVING COATED SURFACES EXHIBITING LOW FRICTION AND METHODS OF REDUCING STICKTION**
MEDIZINISCHE KOMPONENTEN MIT BESCHICHTETEN OBERFLÄCHEN MIT GERINGER REIBUNG UND VERFAHREN ZUR HAFTREIBUNGSVERMINDERUNG
COMPOSANTS MÉDICAUX À SURFACES REVÊTUES À FAIBLE FROTTEMENT ET PROCÉDÉ DE RÉDUCTION DE L'ADHÉRENCE

(30) Priority: 15.09.2006 US 844741 P
(43) Date of publication of application: 27.05.2009
(73) Proprietor: Becton, Dickinson & Company, Franklin Lakes, NJ 07417-0880 (US)
(72) Inventor: WU, Shang-Ren, Mahwah, New Jersey 07430 (US); ALVAREZ, Angel, East Stroudsburg, Pennsylvania 18301 (US); SIMMONS, Stephen, Jr., Ridgewood, New Jersey 07450 (US)
(74) Representative: von Kreisler Selting Werner
(86) International application number: PCT/US2007/078491
(87) International publication number: WO 2008/034058

(56) References cited:
- WO-A-99/47192
- WO-A-2004/064901
- WO-A-2007/115156
- WO-A-2007/115159
- WO-A-2008/034060
- US-A- 5 736 251
- US-A1- 2007 148 326

## Description

### BACKGROUND OF THE INVENTION

### Field of the Invention

This invention relates to medical components having surfaces in sliding engagement, such as syringe assemblies, coated with a composition comprising organopolysiloxane(s) to reduce static and kinetic friction between slidable surfaces, and articles of low friction prepared thereby.

### Description of Related Art

Certain devices require slow and controlled initiation and maintenance of sliding movement of one surface over another surface. It is well known that two stationary surfaces having a sliding relationship often exhibit sufficient resistance to initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose".

"Breakout force" refers to the force required to overcome static friction between surfaces of a syringe assembly that has been previously moved in a sliding relationship, but has been stationary ("parked" or not moved) for a short period of time (for example, milliseconds to hours). A less well known but important frictional force is "breakloose force", which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Breakout and breakloose forces are particularly troublesome in liquid dispensing devices, such as syringes, used to deliver small, accurately measured quantities of a liquid by smooth incremental line to line advancement of one surface over a second surface. The problem is also encountered in devices using stopcocks, such as burets, pipets, addition funnels, and the like where careful dropwise control of flow is desired.

The problems of excessive breakout and breakloose forces are related to friction. Friction is generally defined as the resisting force that arises when a surface of one substance slides, or tends to slide, over an adjoining surface of itself or another substance. Between surfaces of solids in contact, there may be two kinds of friction: (1) the resistance opposing the force required to start to move one surface over another, conventionally known as static friction, and (2) the resistance opposing the force required to move one surface over another at a variable, fixed, or predetermined speed, conventionally known as kinetic friction.

The force required to overcome static friction and induce breakout or breakloose is referred to as the "breakout force" or "breakloose force", respectively, and the force required to maintain steady slide of one surface over another after breakout or breakloose is referred to as the "sustaining force". Two main factors, sticktion and inertia, contribute to static friction and thus to the breakout or breakloose force. The term "stick" or "sticktion" as used herein denotes the tendency of two surfaces in stationary contact to develop a degree of adherence to each other. The term "inertia" is conventionally defined as the indisposition to motion which must be overcome to set a mass in motion. In the context of the present invention, inertia is understood to denote that component of the breakout or breakloose force which does not involve adherence.

Breakout or breakloose forces, in particular the degree of stick, vary according to the composition of the surfaces. In general, materials having elasticity show greater stick than non-elastic materials. The length of time that surfaces have been in stationary contact with each other also influences breakout and/or breakloose forces. In the syringe art, the term "parking" denotes storage time, shelf time, or the interval between filling and discharge. Parking time generally increases breakout or breakloose force, particularly if the syringe has been refrigerated or heated during parking.

WO99/4719 refers to a method for lubricating a sealing member and a medicament chamber in a drug delivery device such as syringes and plungers, including providing lubricating silicone on the surface of the sealing member.

WO2004/064901 discloses a chamber for a medical article, i.e. a syringe having a coating made of organopolysiloxane, in order to reduce frictions and stiction.

US-A-5736251 discloses a lubricious silicone surface modifying treatment and coating for modifying the frictional or slip surface characteristics of shaped elastomeric articles such as syringes and plungers.

A conventional approach to overcoming breakout or breakloose has been application of a lubricant to a surface to surface interface. Common lubricants used are hydrocarbon oils, such as mineral oils, peanut oil, vegetable oils, and the like. Such products have the disadvantage of being soluble in a variety of fluids, such as vehicles commonly used to dispense medicaments. In addition, these lubricants are subject to air oxidation resulting in viscosity changes and objectionable color development. Further, they are particularly likely to migrate from the surface to surface interface. Such lubricant migration is generally thought to be responsible for the increase in breakout or breakloose force with time in parking.

Silicone oils are also commonly used as lubricants, are not subject to oxidation, but migration and stick do occur, and high breakout and/or breakloose forces are a problem. Polytetrafluoroethylene surfaces provide some reduction in breakout and/or breakloose forces, but this material is very expensive, and the approach has not been totally effective.

Thus there is a need for a better system to overcome high breakout and breakloose forces whereby smooth transition of two surfaces from stationary contact into sliding contact can be achieved.

### SUMMARY OF THE INVENTION

The present invention provides a chamber for a medical article, the chamber having an inner surface adapted to sealingly engage a sealing member for a medical article, wherein the inner surface of the chamber has a coating thereon prepared from a composition comprising an organopolysiloxane, the coating being adhered to the inner surface by crosslinking induced by (1) oxidative treatment; and (2) (a) irradiation with an isotope, electron beam or ultraviolet radiation or (b) heat treatment, as mentioned in claim 1.

In other embodiments, the present invention provides a sealing member for a medical article, the sealing member having an exterior surface in sliding engagement with an interior surface of a chamber of a medical article and adapted to sealingly engage the interior surface of the chamber, wherein the exterior surface of the sealing member has a coating thereon prepared from a composition comprising an organopolysiloxane, the coating being adhered to the exterior surface by crosslinking induced by (1) oxidative treatment; and (2) (a) irradiation with an isotope, electron beam or ultraviolet radiation or (b) heat treatment.

### BRIEF DESCRIPTION OF THE DRAWINGS

The present invention will best be understood from the following description of specific embodiments when read in connection with the accompanying drawings:

Fig. 1 is a graph of infusion pump actuation force test results of Comparative Sample Group 1 at a feed rate of 0.1 ml/hr for a syringe assembly having a syringe barrel treated with gamma radiation;

Fig. 2 is a graph of infusion pump actuation force test results of Comparative Sample Group 1 at a feed rate of 1.0 ml/hr for a syringe assembly having a syringe barrel treated with gamma radiation;

Fig. 3 is a graph of infusion pump actuation force test results of Comparative Sample Group 1 at a feed rate of 10 ml/hr for a syringe assembly having a syringe barrel treated with gamma radiation;

Fig. 4 is a graph of infusion pump actuation force test results of Comparative Sample Group 2 at a feed rate of 0.1 ml/hr for a syringe assembly having a syringe barrel treated with oxidative (plasma) treatment;

Fig. 5 is a graph of infusion pump actuation force test results of Comparative Sample Group 2 at a feed rate of 1.0 ml/hr for a syringe assembly having a syringe barrel treated with plasma treatment;

Fig. 6 is a graph of infusion pump actuation force test results of Comparative Sample Group 2 at a feed rate of 10 ml/hr for a syringe assembly having a syringe barrel treated with plasma treatment;

Fig. 7 is a graph of infusion pump actuation force test results of Sample Group 3 at a feed rate of 0.1 ml/hr for a syringe assembly having a syringe barrel treated with plasma treatment and gamma radiation according to the present invention;

Fig. 8 is a graph of infusion pump actuation force test results of Sample Group 3 at a feed rate of 1.0 ml/hr for a syringe assembly having a syringe barrel treated with plasma treatment and gamma radiation according to the present invention;

Fig. 9 is a graph of infusion pump actuation force test results of Sample Group 3 at a feed rate of 10 ml/hr for a syringe assembly having a syringe barrel treated with plasma treatment and gamma radiation according to the present invention;

Fig. 10 is a graph of infusion pump actuation force test results of Comparative Sample Group 4 at a feed rate of 0.1 ml/hr for a syringe assembly having a syringe barrel treated with gamma radiation;

Fig. 11 is a graph of infusion pump actuation force test results of Comparative Sample Group 4 at a feed rate of 1.0 ml/hr for a syringe assembly having a syringe barrel treated with gamma radiation;

Fig. 12 is a graph of infusion pump actuation force test results of Comparative Sample Group 4 at a feed rate of 10 ml/hr for a syringe assembly having a syringe barrel treated with gamma radiation;

Fig. 13 is a graph of infusion pump actuation force test results of Sample Group 5 at a feed rate of 0.1 ml/hr for a syringe assembly having a syringe barrel treated with plasma treatment and gamma radiation according to the present invention;

Fig. 14 is a graph of infusion pump actuation force test results of Sample Group 5 at a feed rate of 1.0 ml/hr for a syringe assembly having a syringe barrel treated with plasma treatment and gamma radiation according to the present invention;

Fig. 15 is a graph of infusion pump actuation force test results of Sample Group 5 at a feed rate of 10 ml/hr for a syringe assembly having a syringe barrel treated with plasma treatment and gamma radiation according to the present invention;

Fig. 16 is a graph of infusion pump actuation force test results of Comparative Sample Group 6 at a feed rate of 0.1 ml/hr for a syringe assembly having a syringe barrel treated with gamma radiation;

Fig. 17 is a graph of infusion pump actuation force test results of Comparative Sample Group 6 at a feed rate of 1.0 ml/hr for a syringe assembly having a syringe barrel treated with gamma radiation;

Fig. 18 is a graph of infusion pump actuation force test results of Comparative Sample Group 6 at a feed rate of 10 ml/hr for a syringe assembly having a syringe barrel treated with gamma radiation;

Fig. 19 is a graph of infusion pump actuation force test results of Sample Group 7 at a feed rate of 0.1 ml/hr for a syringe assembly having a syringe barrel treated with plasma treatment and gamma radiation according to the present invention;

Fig. 20 is a graph of infusion pump actuation force test results of Sample Group 7 at a feed rate of 1.0 ml/hr for a syringe assembly having a syringe barrel treated with plasma treatment and gamma radiation according to the present invention; and

Fig. 21 is a graph of infusion pump actuation force test results of Sample Group 7 at a feed rate of 10 ml/hr for a syringe assembly having a syringe barrel treated with plasma treatment and gamma radiation according to the present invention.

### DETAILED DESCRIPTION OF THE INVENTION

At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical values, however, inherently contain certain errors necessarily resulting from the standard deviation found in their respective testing measurements. Furthermore, when numerical ranges of varying scope are set forth herein, it is contemplated that any combination of these values inclusive of the recited values may be used.

Also, it should be understood that any numerical range recited herein is intended to include all sub-ranges subsumed therein. For example, a range of "1 to 10" is intended to include all sub-ranges between and including the recited minimum value of 1 and the recited maximum value of 10, that is, having a minimum value equal to or greater than 1 and a maximum value of equal to or less than 10.

In some embodiments, the present invention encompasses a component for a medical article having a surface coated with an organopolysiloxane. The coated surface of the component is adapted to sealingly engage an adjoining surface of another component of the medical article. For example, the component can comprise a chamber having an inner surface adapted to sealingly engage an exterior surface of a sealing member for a medical article.

In other embodiments, the present invention encompasses a sealing member for a medical article having an exterior surface coated with an organopolysiloxane.

In some embodiments, both the inner surface of the chamber and the exterior surface of the sealing member are coated with organopolysiloxanes, which can be the same or different.

The coating on the surface(s) of the chamber and/or sealing member is subjected to (1) oxidative treatment and (2) irradiation or heat treatment, as discussed in detail below.

The respective surfaces of the inner surface of the chamber and the exterior surface of the sealing member can be in frictional engagement. When used in a medical article, the effects of the present invention can reduce the force required to achieve breakout, breakloose and/or sustaining forces, whereby transition of surfaces from stationary contact to sliding contact occurs without a sudden surge. When breakout or breakloose is complete and the surfaces are in sliding contact, they slide smoothly upon application of very low sustaining force. The effect achieved by the methods of the present invention can be of long duration, and articles, such as syringes, can retain the advantages of low breakout, low breakloose and low sustaining forces for several years. When the chamber is part of a liquid dispensing device, small highly accurate increments of liquid may be dispensed repeatedly without sudden surges. Thus, a syringe including a chamber and/or sealing member treated according to the present invention can be used to administer a medicament to a patient without the danger of surges whereby accurate control of dosage and greatly enhanced patient safety are realized.

As used herein, "medical article" means an article or device that can be useful for medical treatment. Non-limiting examples of medical articles include syringe assemblies, drug cartridges, needleless injectors, liquid dispensing devices and liquid metering devices. In some embodiments, the medical article is a syringe assembly comprising a syringe chamber or barrel (for receiving water, saline or a medicament, for example) and a sealing member.

The chamber is formed from cyclic polyolefin. For example, the polyolefin can be a homopolymer or a copolymer of an aliphatic monoolefin, the aliphatic monoolefin preferably having 2 to 6 carbon atoms, such as polypropylene. In some embodiments, the polyolefin can be basically linear, but optionally may contain side chains such as are found, for instance, in conventional, low density polyethylene. In some embodiments, the polyolefin is at least 50% isotactic. In other embodiments, the polyolefin is at least about 99% isotactic in structure. In some embodiments, syndiotactic polymers can be used. Non-limiting examples of suitable cyclic polyolefins include norbornene polymers such as are disclosed in U.S. Patents Nos. 6,525,144, 6,511,756, 5,599,882, and 5,034,482 (each of Nippon Zeon), 7,037,993, 6,995,226, 6,908,970, 6,653,424 and 6,486,264 (each of Zeon Corp.), 7,026,401, and 6,951,898 (Ticona), 6,063,886 (Mitsui Chemicals), 5,866,662, 5,856,414, 5,623,039 and 5,610,253 (Hoechst), 5,854,349, and 5,650,471 (Mitsui Petrochemical and Hoechst) and as described in "Polycyclic olefins", process Economics Program (July 1998) SRI Consulting. Non-limiting examples of suitable cyclic polyolefins include Apel™ cyclic polyolefins available from Mitsui Petrochemical, Topas™ cyclic polyolefins available from Ticona Engineering Polymers, Zeonor™ or Zeonex™ cyclic polyolefins available from Zeon Corporation, and cyclic polyolefins available from Promerus LLC.

The polyolefin can contain a small amount, generally from 0.1 to 10 percent, of an additional polymer incorporated into the composition by copolymerization with the appropriate monomer. Such copolymers may be added to the composition to enhance other characteristics of the final composition, and may be, for example, polyacrylate, polystyrene and the like.

In some embodiments, the chamber may be constructed of a polyolefin composition which includes a radiation stabilizing additive to impart radiation stability to the container, such as a mobilizing additive which contributes to the radiation stability of the container, such as for example those disclosed in U.S. Patent Nos. 4,959,402 and 4,994,552, assigned to Becton, Dickinson and Company.

The other component of the medical article in contact with the chamber is the sealing member. The sealing member can be formed from any elastomeric or plastic material. Elastomers are used in many important and critical applications in medical devices and pharmaceutical packaging. As a class of materials, their unique characteristics, such as flexibility, resilience, extendability, and sealability, have proven particularly well suited for products such as catheters, syringe tips, drug vial articles, tubing, gloves and hoses. Three primary synthetic thermoset elastomers typically are used in medical applications: polyisoprene rubber, silicone rubber, and butyl rubber. Of the three rubbers, butyl rubber has been the most common choice for articles due to its high cleanness and permeation resistance which enables the rubber to protect oxygen- and water-sensitive drugs.

Suitable butyl rubbers useful in the method of the present invention include copolymers of isobutylene ( 97-98%) and isoprene ( 2-3%). The butyl rubber can be halogenated with chlorine or bromine. Suitable butyl rubber vulcanizates can provide good abrasion resistance, excellent impermeability to gases, a high dielectric constant, excellent resistance to aging and sunlight, and superior shock-absorbing and vibration-damping qualities to articles formed therefrom. Non-limiting examples of suitable rubber stoppers include those available from West Pharmaceuticals, American Gasket Rubber, Stelmi and Helvoet Rubber & Plastic Technologies BV.

Other useful elastomeric copolymers include, without limitation, thermoplastic elastomers, thermoplastic vulcanizates, styrene copolymers such as styrene-butadiene (SBR or SBS) copolymers, styrene-isoprene (SIS) block polymers or styrene-isoprene/butadiene (SIBS), in which the content of styrene in the styrene block copolymer ranges from 10% to 70%, and preferably from 20% to 50%. Non-limiting examples of suitable styrene-butadiene stoppers are available from Firestone Polymers, Dow, Reichhold, Kokoku Rubber Inc. and Chemix Ltd. Other suitable thermoplastic elastomers are available from GLS, Tecknor Apex, AES, Mitsubishi and Solvay Engineered Polymers, for example. The elastomer composition can include, without limitation, antioxidants and/or inorganic reinforcing agents to preserve the stability of the elastomer composition.

In some embodiments, the sealing member can be a stopper, O-ring, plunger tip, or piston, for example. Syringe plunger tips or pistons typically are made of a compressible, resilient material such as rubber, because of the rubber's ability to provide a seal between the plunger and interior housing of the syringe. Syringe plungers, like other equipment used in the care and treatment of patients, have to meet high performance standards, such as the ability to provide a tight seal between the plunger and the barrel of the syringe.

In some embodiments, the respective surfaces of the inner surface of the chamber and the exterior surface of the sealing member can be optionally subjected to oxidative treatment, for example plasma treatment, before coating as discussed in detail below. The plasma treatment may be carried out in any common vacuum or atmospheric plasma generation equipment. Any suitable ionizing plasma may be used, as, for example, a plasma generated by a glow discharge or a corona discharge. The plasma may be generated from a variety of gases or mixtures thereof, such as air, hydrogen, helium, ammonia, nitrogen, oxygen, neon, argon, krypton and xenon. Similar oxidative treatment conditions, such as gas pressures, equipment and power settings, etc., are discussed in detail below with respect to oxidative treatment of the coated substrate.

The coating is applied to at least a portion of the sliding surface(s) of the chamber and/or sealing member. In some embodiments, the chamber is coated with the coating described below and the sealing member is uncoated or coated with a polydimethylsiloxane coating. In other embodiments, the sealing member is coated with the coating described below and the chamber is uncoated or coated with a polydimethylsiloxane coating. In other embodiments, both the chamber and sealing member are coated with coatings as described below. Methods for coating the surface(s) are discussed in detail below.

The chamber and/or sealing member can be coated with a coating prepared from a composition comprising one or more organopolysiloxane(s). In some embodiments, the organopolysiloxane has a viscosity ranging from 0.1 - 1000 Pa·s (100 to 1,000,000 centistokes (cst)), prior to any curing step. In some embodiments, the organopolysiloxane has a viscosity ranging from 1.0 - 100 Pa·s (1,000 centistokes to 100,000 centistokes (cst)). In some embodiments, the organopolysiloxane has a viscosity ranging from 1.0 - 15 Pa·s (1,000 cst to 15,000 cst). In other embodiments, the organopolysiloxane has a viscosity of about 12.5 Pa·s (12,500 cst). The viscosity can be measured using a Brookfield DV II+ viscometer.

The coating is adhered to the inner surface of the chamber and/or sealing member by crosslinking induced by (1) oxidative treatment and (2) (a) irradiation with an isotope, electron beam or ultraviolet radiation or (b) heat treatment, as discussed below.

It is believed that the oxidative treatment induces cross-linking in the organopolysiloxane, whereby the organopolysiloxane is converted to a high molecular weight three dimensional polymer network. It is further believed that this oxidative treatment can also induce crosslinking of the organopolysiloxane to the surface(s). It is further believed that the subsequent heat treatment or radiation treatment promotes additional crosslinking.

In some embodiments, the organopolysiloxane comprises an alkyl-substituted organopolysiloxane, for example as is represented by the following structural formula (I): 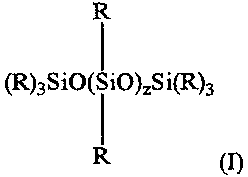 wherein R is alkyl and Z is 30 to 4,500. In some embodiments, the organopolysiloxane of Formula (I) can be represented by the following structural formula (II): 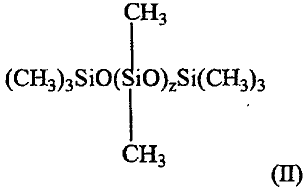 wherein Z can be as above, or for example can be 300 to 2,000, 300 to 1800, or 300 to 1,350. In some embodiments, the organopolysiloxane is a polydimethylsiloxane, such as DOW CORNING® 360 polydimethylsiloxane or NUSIL polydimethylsiloxane having a viscosity ranging from 0.1 - 1000 Pa·s (100 to 1,000,000 cst).

In other embodiments, the organopolysiloxane comprises one or more curable or reactive functional groups, such as alkenyl groups. As used herein, the term "cure" as used in connection with a composition, i.e., a "cured composition" or a "cured coating" shall mean that at least a portion of the crosslinkable components which form the composition are at least partially crosslinked. As used herein, the term "curable", as used in connection with a component of the composition, means that the component has functional groups capable of being crosslinked, for example alkenyl groups such as vinyl groups. In certain embodiments of the present invention, the crosslink density of the crosslinkable components, i.e., the degree of crosslinking, ranges from 5% to 100% of complete crosslinking. One skilled in the art will understand that the presence and degree of crosslinking, i.e., the crosslink density, can be determined by a variety of methods, such as dynamic mechanical thermal analysis (DMTA). This method determines the glass transition temperature and crosslink density of free films of coatings or polymers. These physical properties of a cured material are related to the structure of the crosslinked network.

In some embodiments, the organopolysiloxane comprises at least one alkenyl group. Each alkenyl group can be independently selected from the group consisting of vinyl, allyl, propenyl, butenyl, pentenyl, hexenyl, heptenyl, octenyl, nonenyl, and decenyl. One skilled in the art would understand that the organopolysiloxane can comprise one or more of any of the above types of alkenyl groups and mixtures thereof In some embodiments, at least one alkenyl group is vinyl. Higher alkenyl or vinyl content provides more efficient crosslinking.

In some embodiments, the organopolysiloxane can be represented by the following structural formulae (III) or (IV): 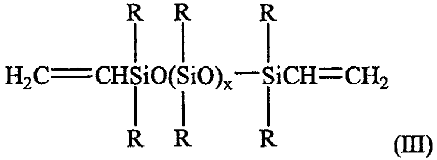 or 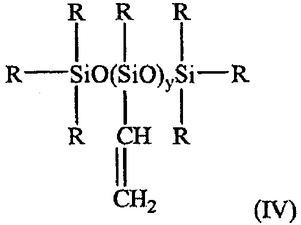 wherein R is alkyl, haloalkyl, aryl, haloaryl, cycloalkyl, silacyclopentyl, aralkyl, and mixtures thereof; X is 60 to 1000, preferably 200 to 320; and y is 3 to 25. Copolymers and mixtures of these polymers are also contemplated.

Non-limiting examples of useful vinyl functional organopolysiloxanes include: vinyldimethylsiloxy terminated polydimethylsiloxanes; trimethylsiloxy terminated vinylmethyl, dimethylpolysiloxane copolymers; vinyldimethylsiloxy terminated vinylmethyl, dimethylpolysiloxane copolymers; divinylmethylsiloxy terminated polydimethylsiloxanes; vinyl, n-butylmethyl terminated polydimethylsiloxanes; and vinylphenylmethylsiloxy terminated polydimethylsiloxanes.

In some embodiments, a mixture of siloxane polymers selected from those of Formulae II, III and/or IV can be used. For example, the mixture can comprise two different molecular weight vinyldimethylsiloxy terminated polydimethylsiloxane polymers, wherein one of the polymers has an average molecular weight of 1,000 to 25,000 and preferably about 16,400, and the other polymer has an average molecular weight of 30,000 to 71,000 and preferably about 38,000. Generally, the lower molecular weight siloxane can be present in amounts of 20% to 80%, such as about 60% by weight of this mixture; and the higher molecular weight siloxane can be present in amounts of 80% to 20%, such as about 40% by weight of this mixture.

Another non-limiting example of a suitable vinyl functional organopolysiloxane is (7.0-8.0% vinylmethylsiloxane) - dimethylsiloxane copolymer, trimethylsiloxy terminated, such as VDT-731 vinylmethylsiloxane copolymer which is commercially available from Gelest, Inc. of Morrisville, PA.

In some embodiments, the organopolysiloxane can comprise at least two polar groups. Each polar group can be independently selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, and carboxypropyl groups. One skilled in the art would understand that the organopolysiloxane can comprise one or more of any of the above polar groups and mixtures thereof

In some embodiments, the polar groups are acrylate groups, for example acryloxypropyl groups. In other embodiments, the polar groups are methacrylate groups, such as methacryloxypropyl groups.

The organopolysiloxane having polar groups can further comprise one or more alkyl groups and/or aryl groups, such as methyl groups, ethyl groups, or phenyl groups.

Non-limiting examples of such organopolysiloxanes include [15-20% (acryloxypropyl)methylsiloxane] - dimethylsiloxane copolymer, such as UMS-182 acrylate functional siloxane, which is available from Gelest, Inc. of Morrisville, PA, and SILCOLEASE® PC970 acrylated silicone polymer, which is available from Rhodia-Silicones.

In other embodiments, such an organopolysiloxane can be represented by the formula (V): 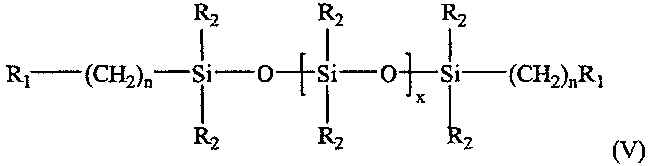 wherein R₁ is selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, carboxypropyl, and fluoro groups; and R₂ is alkyl, n ranges from 2 to 4, and x is an integer sufficient to give the lubricant a viscosity of 0.1 - 1000 Pa·s (100 to 1,000,000 cst).

While not wishing to be bound by any theory, it is believed that the polar siloxanes may help reduce the coefficient of friction between the engaged surfaces. Also, after irradiation, it is believed that the viscosity of the polar siloxane may increase and improve the adhesion of the coating to substrate.

In some embodiments, the organopolysiloxane can further comprise one or more fluoro groups, such as -F or fluoroalkyl groups such as trifluoromethyl groups. Other useful organopolysiloxanes include polyfluoroalkylmethyl siloxanes and fluoroalkyl, dimethyl siloxane copolymers.

In some embodiments, the composition can further comprise one or more cyclic siloxane(s), for example, octamethylcyclotetrasiloxane and/or decamethylcyclopentasiloxane.

In some embodiments, the organopolysiloxane can be represented by the following structural formula (VI): 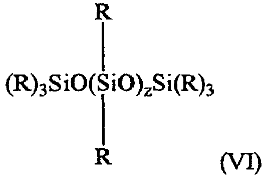 wherein R is haloalkyl, aryl (such as phenyl), haloaryl, cycloalkyl, silacyclopentyl, aralkyl and mixtures thereof; and Z is 20 to 1,800.

In some embodiments, the organopolysiloxane comprises at least two pendant hydrogen groups. Non-limiting examples of suitable organopolysiloxanes comprising at least two pendant hydrogen groups include organopolysiloxanes having pendant hydrogen groups along the polymer backbone or terminal hydrogen groups. In some embodiments, the organopolysiloxane can be represented by the following structural formulae (VII): 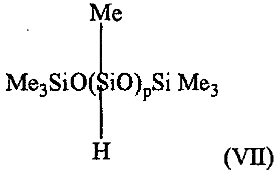 wherein p is 8 to 125, for example, about 30. In other embodiments, the organopolysiloxane can be represented by the following structural formula (VIII):

HMe2SiO(MeZSiO)ₚSiMe₂H (VIII)

wherein p is 140 to 170, for example, 150 to 160. A mixture of these polymers can be used comprising two different molecular weight materials. For example, 2% to 5% by weight of the mixture of a trimethylsiloxy terminated polymethylhydrosiloxane having an average molecular weight of 400 to 7,500, for example about 1900, can be used in admixture with 98% to 95% of a dimethylhydro siloxy-terminated polydimethylsiloxane having an average molecular weight of 400 to 37,000 and preferably about 12,000. Non-limiting examples of useful organopolysiloxanes comprising at least two pendant hydrogen groups include dimethylhydro terminated polydimethylsiloxanes; methylhydro, dimethylpolysiloxane copolymers; dimethylhydrosiloxy terminated methyloctyl dimethylpolysiloxane copolymers; and methylhydro, phenylmethyl siloxane copolymers.

In some embodiments, the composition comprises hydroxy functional siloxanes, for example a hydroxy functional siloxane comprising at least two hydroxyl groups, such as for example: 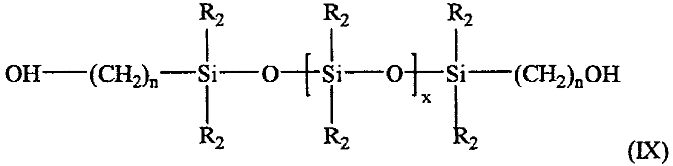 wherein R₂ is alkyl, n ranges from 0 to 4, and x is an integer sufficient to give the lubricant a viscosity of 0.1 - 1000 Pa·s (100 to 1,000,000 cst). In some embodiments, moisture-curable siloxanes which have moisture-curing character as a result of functionality include siloxanes having functional groups such as: alkoxy; aryloxy; oxime; epoxy; -OOCR; N,N-dialkylamino; N,N-dialkylaminoxy; N-alkylamido; -O-NH-C(O)-R; -O-C(=NCH₃)-NH-CH₃; and -O-C(CH₃)=CH₂, wherein R is H or hydrocarbyl. As used herein, "moisture-curable" means that the siloxane is curable at ambient conditions in the presence of atmospheric moisture.

Mixtures of one or more of the organopolysiloxanes discussed above can be used in the present invention.

In some embodiments, the organopolysiloxane comprises 90 to 100 weight percent of the composition. In other embodiments, the organopolysiloxane comprises 95 to 100 weight percent of the composition. In other embodiments, the organopolysiloxane comprises 100 weight percent of the composition.

In some embodiments, the composition further comprises a catalytic amount of a catalyst for promoting crosslinking of crosslinkable groups of the organopolysiloxane(s). Non-limiting examples of suitable catalysts for promoting ultraviolet radiation cure include any suitable photoinitiator which is capable of initiating polymerization of the reactive silicone polymer upon exposure to UV light Non-limiting examples of useful UV light-induced polymerization photoinitiators include ketones such as benzyl and benzoin, and acyloins and acyloin ethers, such as alpha-hydroxy ketones. Non-limiting examples of available products include IRGACURE 184 (1-hydroxycyclohexyl phenyl ketone), IRGACURE 907 (2-methyl-1-[4-(methylthio)phenyl]-2-(4-morpholinyl)-1-propanone), IRGACURE 369 (2-benzyl-2-N,N-dimethylamino-1-(4-morpholinophenyl)-1-butanone), IRGACURE 500 (the combination of 50% 1-hydroxy cyclohexyl phenyl ketone and 50% benzophenone), IRGACURE 651 (2,2-dimethoxy-1,2-diphenylethan-1-one), IRGACURE 1700 (the combination of 25% bis(2,6-dimethoxybenzoyl-2,4-, 4-trimethyl pentyl) phosphine oxide and 75% 2-hydroxy-2-methyl-1-phenyl-propan-1-one), DAROCUR 1173 (2-hydroxy-2-methyl-1-phenyl-propan-1-one), and DAROCUR 4265 (the combination of 50% 2,4,6-trimethylbenzoyldiphenyl-phosphine oxide and 50% 2-hydroxy-2-methyl-1-phenyl-propan-1-one), all of which are available from CIBA Corp., Tarrytown, N.Y.; and SARCURE SR-1121 (2-hydroxy-2-methyl-1-phenyl propanone) and ESACURE KIP-100F (a mixture of polymeric photoinitiators in 2-hydroxy-2-methyl-1-phenylpropan-1-one), both of which are available from Sartomer, Inc. of Exton, Pa. Of course, mixtures of different photoinitiators may also be used. The photoinitiator is desirably in a liquid form to ensure appropriate mixing and distribution within the composition, although solid photoinitiators may also be used, provided that they are soluble in organopolysiloxane to provide the composition as a homogeneous fluid. The photoinitiator should be present in an amount sufficient to provide the desired rate of photopolymerization, dependent in part on the light source and the extinction coefficient of the photoinitiator. Typically, the photoinitiator components will be present at a total weight of 0.01 to 10%, more preferably from 0.1 to 5%, based on the total weight of the composition.

Non-limiting examples of suitable catalysts for promoting heat cure include platinum or rhodium group metal catalysts, such as Karstedt catalyst Pt₂{[(CH₂=CH)Me₂Si]₂O}₃ or peroxide catalysts, such as dicumyl peroxide The catalyst can be present in an amount ranging from 0.001 to 0.05 weight percent of the composition.

The components of the composition can be formulated in a single composition or two compositions that are mixed prior to application, for example, to separate a catalyst from crosslinkable components until shortly before application. A non-limiting example of a suitable two component composition is a two-part LSR silicone composition commercially available from GE Silicones.

Application of a coating to the inner surface of the chamber or outer surface of the sealing member may be accomplished by any suitable method, as, for example, dipping, brushing, spraying and the like. The composition may be applied neat or it may be applied in a solvent, such as low molecular weight silicone, non-toxic chlorinated or fluorinated hydrocarbons, for example, 1,1,2-trichloro-1,2,2-trifluoroethane, freon or conventional hydrocarbon solvents such as alkanes, toluene, petroleum ether and the like where toxicology is not considered important. The solvent is subsequently removed by evaporation. The coating may be of any convenient thickness and, in practice, the thickness will be determined by such factors as the quantity applied, viscosity of the lubricant and the temperature of application. For reasons of economy, the coating preferably is applied as thinly as practical, since no significant advantage is gained by thicker coatings. The exact thickness of the coating does not appear to be critical and very thin coatings, i.e., one or two microns exhibit effective lubricating properties. While not necessary for operability, it is desirable that the thickness of the coating be substantially uniform throughout.

The coating can be partially or fully crosslinked after application or partially crosslinked to attach to the substrate, and then fully crosslinked at a later time.

The coated chamber and/or coated sealing member is subjected to oxidative treatment, for example plasma treatment. The plasma treatment may be carried out in any common vacuum or atmospheric plasma generation equipment. Any suitable ionizing plasma may be used, as, for example, a plasma generated by a glow discharge or a corona discharge. The plasma may be generated from a variety of gases or mixtures thereof. Gases frequently used include air, hydrogen, helium, ammonia, nitrogen, oxygen, neon, argon, krypton and xenon. Any gas pressure may be used, for example, atmospheric pressure or 5 mm of Hg or below, such as 0.1 to 1.0 mm of Hg. In some embodiments such as atmospheric oxidative methods, the ionizing plasma is introduced directly from a small port in the chamber or through the opening later sealed by the sealing member. The external surface of the coated sealing member can be treated directly similarly to current corona or plasma treatment methods. In other embodiments, such as vacuum based equipment, the plasma can be excited around the coated sealing member or coated chamber and allowed to diffuse into the chamber and sealing member features. Alternatively, the plasma may be excited within the interior of the open chamber by properly controlling electrode position.

A wide range of power settings, radio frequencies and durations of exposure of the lubricant and surface to the plasma may be used. Non-limiting ranges for these three parameters which provide advantageous results are DC or AC power levels up to 200 watts, from 0.1 to 50 megahertz and from 1 second to 30 minutes, respectively.

After oxidative treatment, the treated chamber and/or treated sealing member is subjected to heat treatment or irradiation with an isotope (such as gamma radiation), electron beam or ultraviolet radiation. Alternatively, the treated chamber and/or treated sealing member can be heat treated via oven or radio frequency (RF). In the case of oven crosslinking, temperatures can range from 120° to 140° C and residence time in the oven is generally 30 to 40 seconds, depending on the precise formulation. If RF techniques are used, the coil should conduct enough heat to obtain a substrate surface temperature of 150° to 200°C. At these temperatures, only 2 to 4 seconds are required for cure.

In some embodiments, the coating is at least partially crosslinked by irradiation with an isotope, electron beam or ultraviolet radiation. This technique has the advantage of sterilizing as well, which is useful in medical applications. Radiation sterilization in the form of ionizing radiation commonly is used in hospitals for medical devices such as catheters, surgical items, and critical care tools. Gamma irradiation is the most popular form of radiation sterilization and typically is used when materials are sensitive to the heat sterilization processes such as autoclaving but are compatible with ionizing radiation. Gamma irradiation exerts a microbicidal effect by oxidizing biological tissue, and thus provides a simple, rapid and efficacious method of sterilization. Gamma rays are used either from a cobalt-60 (⁶⁰Co) isotope source or from a machine-generated accelerated electron source. Sufficient exposures are achieved when the materials to be sterilized are moved around an exposed ⁶⁰Co source for a defined period of time. The most commonly used dose for sterilizing medical articles is 5 to 100 kGy, for example, 5-50 kGy.

In some embodiments, a surface lubricant layer 0.3 to 10, preferably 0.8 to 4.0 microns thick may be applied over the crosslinked organopolysiloxane coating described above. The surface lubricant can be conventional silicone oil (organopolysiloxane) of viscosity 0.1 - 1000, 0.1-60 Pa·s (100 to 1,000,000, 100 to 60,000) or preferably 1.0 - 12.5 Pa·s (1000 to 12,500 cst). The surface lubricating layer may be applied by any of the conventional methods described above. The preferred methods for applying the surface lubricant are by spraying or dipping the syringe barrel into a solution, about 4% by weight, of the surface lubricant in a solvent such as chloroform, dichloromethane or preferably a chlorofluorocarbon, such as FREON™ TF. The surface lubricant may optionally be lightly crosslinked by oxidative treatment and/or radiation.

In some embodiments in which both the chamber and sealing member are coated with organopolysiloxanes, the viscosity of the organopolysiloxane coating the chamber can be greater than the viscosity of the organopolysiloxane coating the sealing member For example, the viscosity of the organopolysiloxane coating the chamber can be 12.5 Pa·s (12,500 cst), while the viscosity of the organopolysiloxane coating the sealing member can be 1.0 Pa·s (1,000 cst). In other embodiments, the viscosity of the organopolysiloxane coating the chamber can be equal to or less than the viscosity of the organopolysiloxane coating the sealing member. For example, the viscosity of the organopolysiloxane coating the chamber can be 12.5 Pa·s (12,500 cst), while the viscosity of the organopolysiloxane coating the sealing member can be 100 Pa·s (100,000 cst.).

In some embodiments, the coated articles are subjected to a sterilization treatment. Many sterilization techniques are available today to sterilize medical devices to eliminate living organisms such as bacteria, yeasts, mold and viruses. Commonly used sterilization techniques used for medical devices include autoclaving, ethylene oxide (EtO) or gamma irradiation, as well as more recently introduced systems that involve low-temperature gas plasma and vapor phase sterilants.

One common sterilization technique is steam sterilization or autoclaving, which is a relatively simple process that exposes an article, for example, to saturated steam at temperatures of over 120°C for a minimum of twenty minutes at a pressure of about 120 kPa. The process is usually carried out in a pressure vessel designed to withstand the elevated temperature and pressure to kill microorganisms by destroying metabolic and structural components essential to their replication. Autoclaving is the method of choice for sterilization of heat-resistant surgical equipment and intravenous fluid as it is an efficient, reliable, rapid, relatively simple process that does not result in toxic residues.

The present invention is more particularly described in the following examples, which are intended to be illustrative only, as numerous modifications and variations therein will be apparent to those skilled in the art.

### EXAMPLE

Syringe barrels for 10 and 50 ml syringes were coated with a coating composition according to the present invention. The syringe barrels were formed from a cyclic polyolefin. The interior surface of each barrel was coated with DC 360 polydimethylsiloxane having a viscosity of 12,500 cst, available from Dow Coming. Selected barrels were pretreated with argon plasma from the injection end of the barrel for the time period specified in Table 1. The plasma treatment was conducted using a conventional atmospheric plasma treatment apparatus as discussed above.

Helvoet FM460 (Butyl-A) and FM457 (Butyl-B) butyl rubber and Kokoku SBR syringe stoppers were coated with a conventional polydimethylsiloxane having a viscosity of 100,000 cst.

The syringe barrels and stoppers were irradiated using gamma radiation at dosages specified in Table 1.

Each syringe was assembled and filled with 10 or 50 ml of deionized water and autoclaved at 124°C for 30 minutes.

Breakout forces, breakloose forces and sustaining forces may be conveniently measured on a universal mechanical tester or on a testing machine of the type having a constant rate of cross-head movement, as described in detail below. The syringe assemblies were evaluated for breakloose force according to ISO 7886-1 Annex G. The breakloose and sustaining force (in kilograms) of each sample syringe was determined by an Instron Series 5500 at a displacement rate of 100 mm/min according to ISO 7886. The breakloose force is visually determined as the highest peak of the curve or point of where the slope of the curve changes on the graph. The sustaining force is the average force for the stopper to move an additional 45 mm for the 10 ml barrel and 85 mm for the 50 ml barrel after breakloose. The breakloose and sustaining values reported in Table 1 below are the results of two samples for each of Sample Groups 1-12.

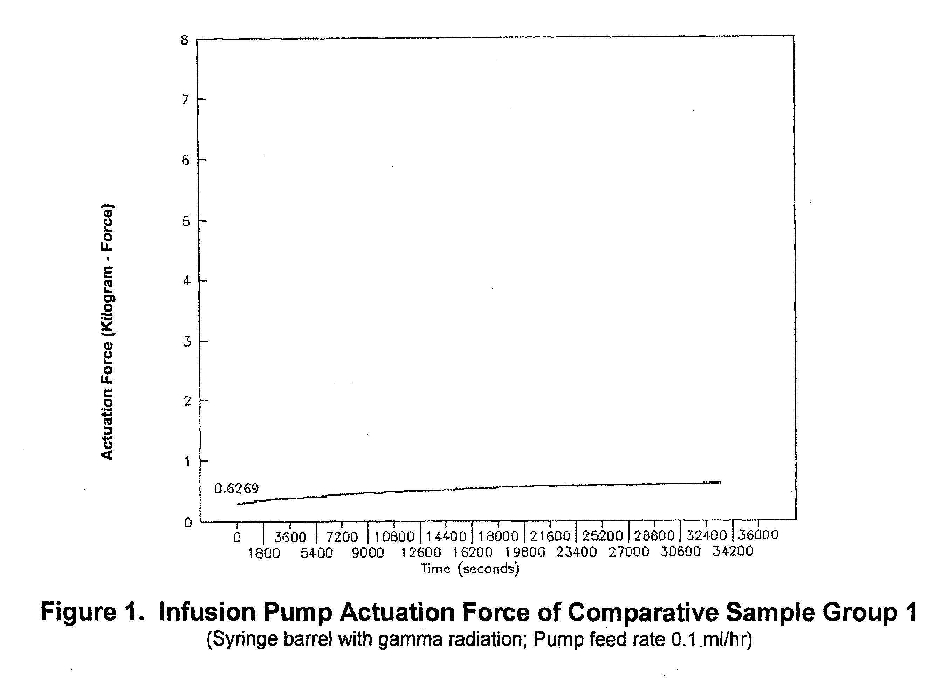

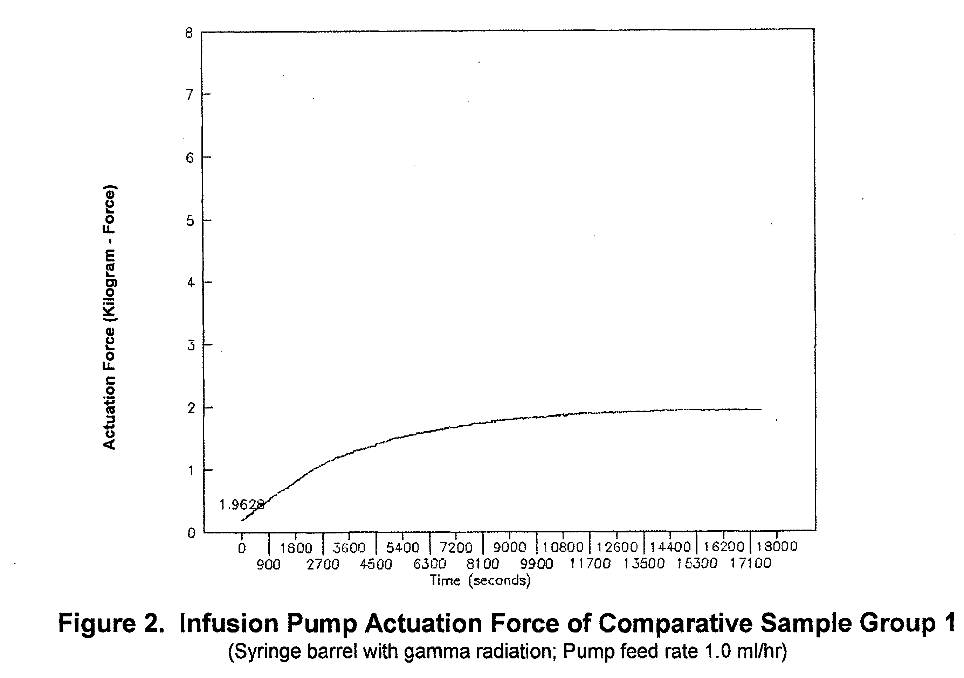

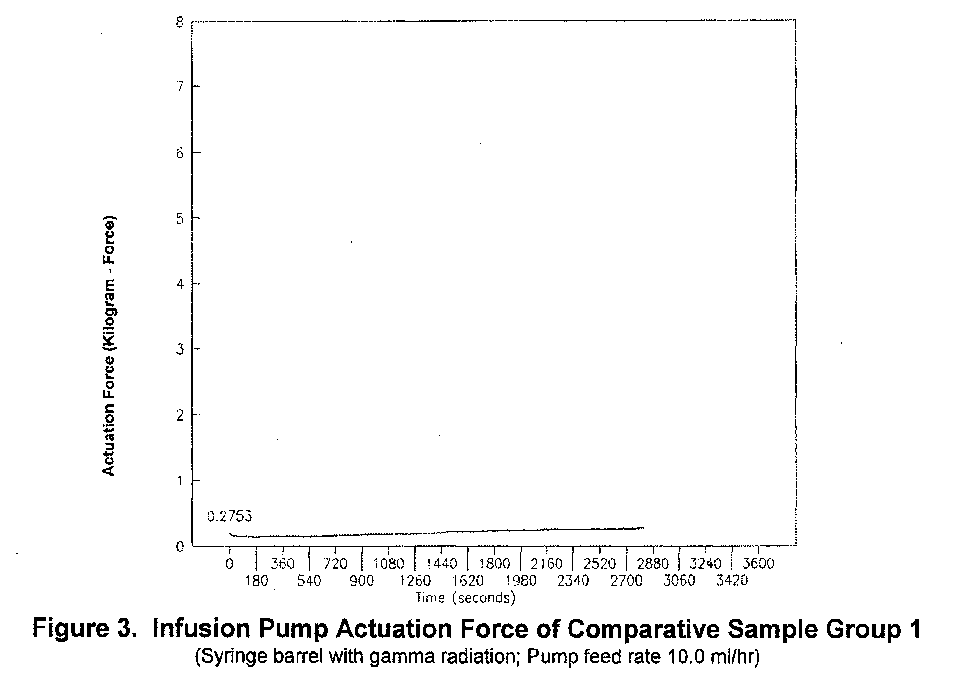

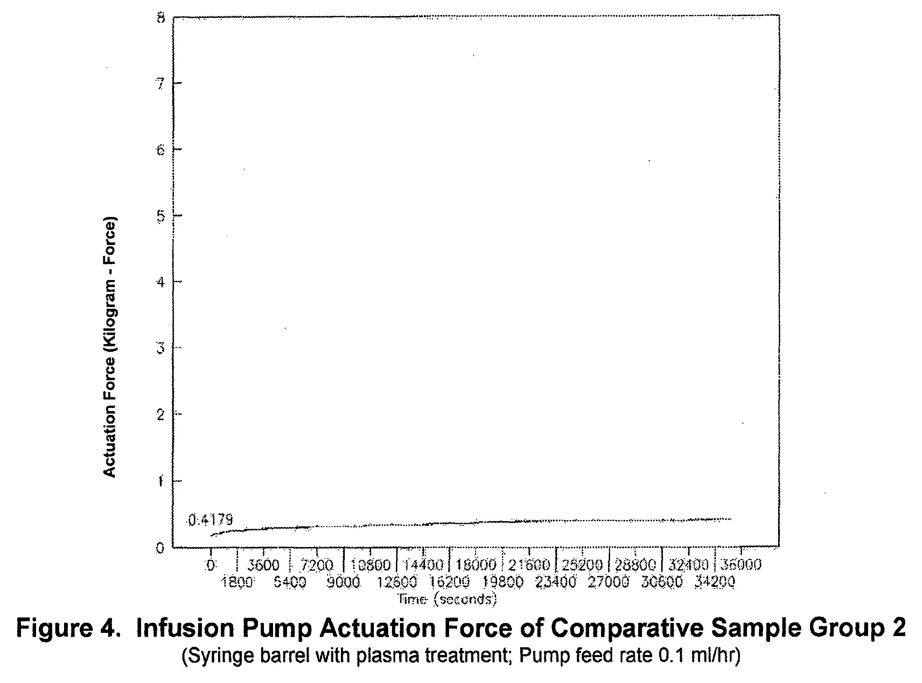

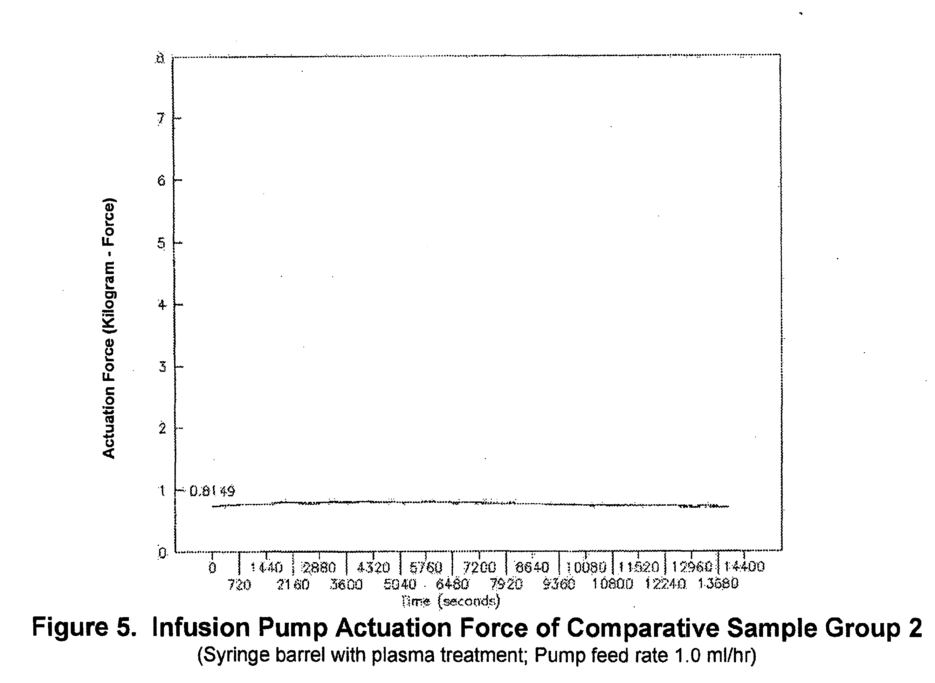

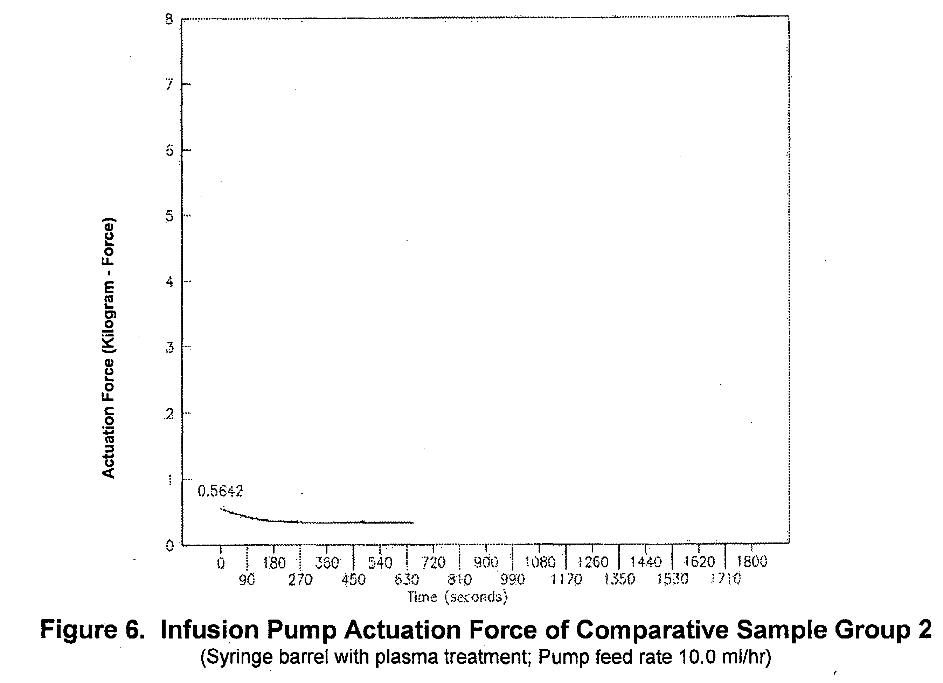

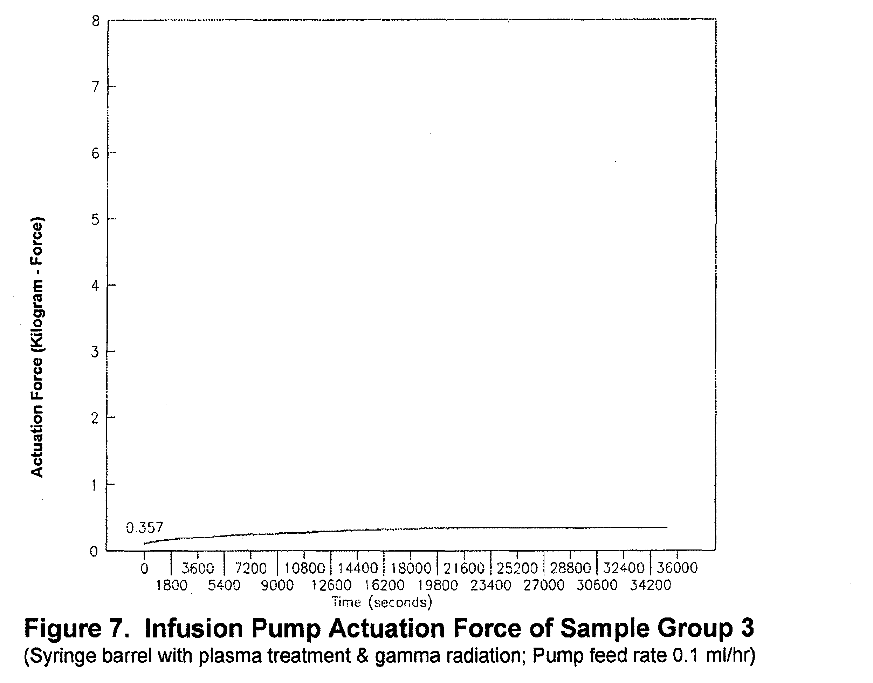

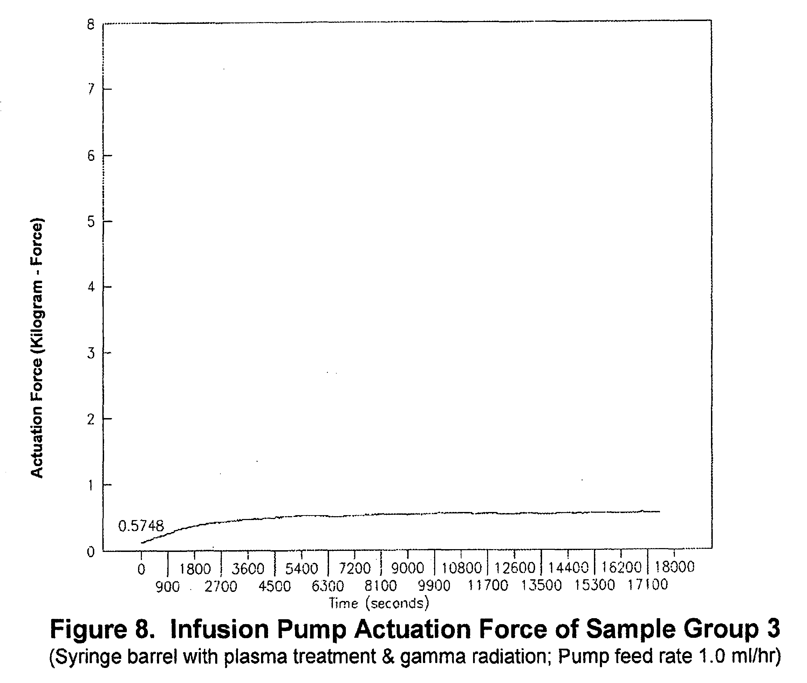

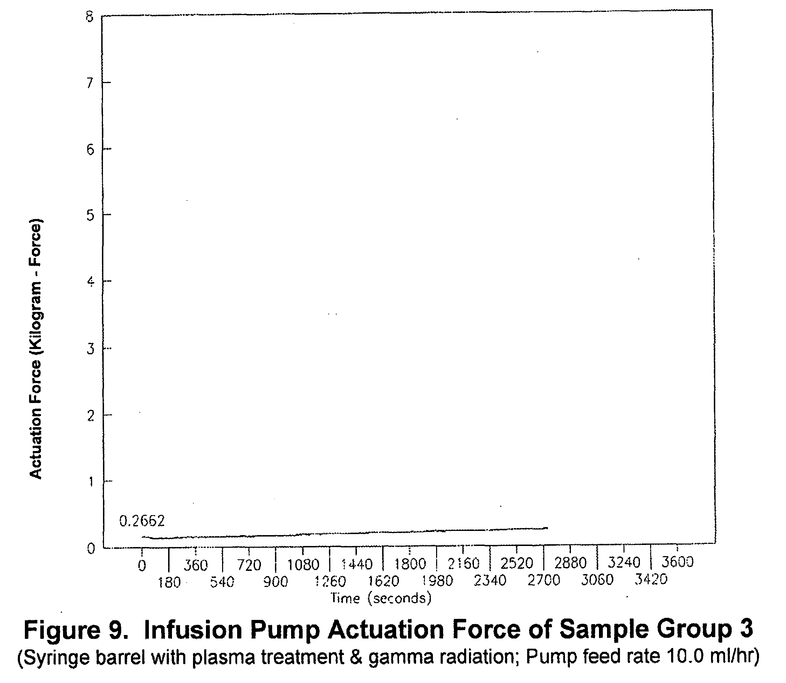

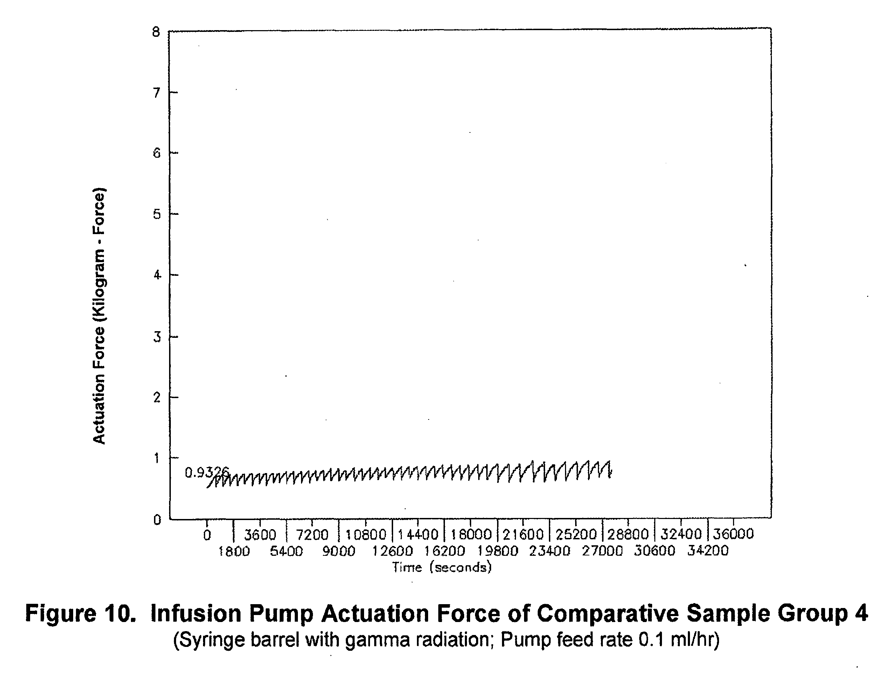

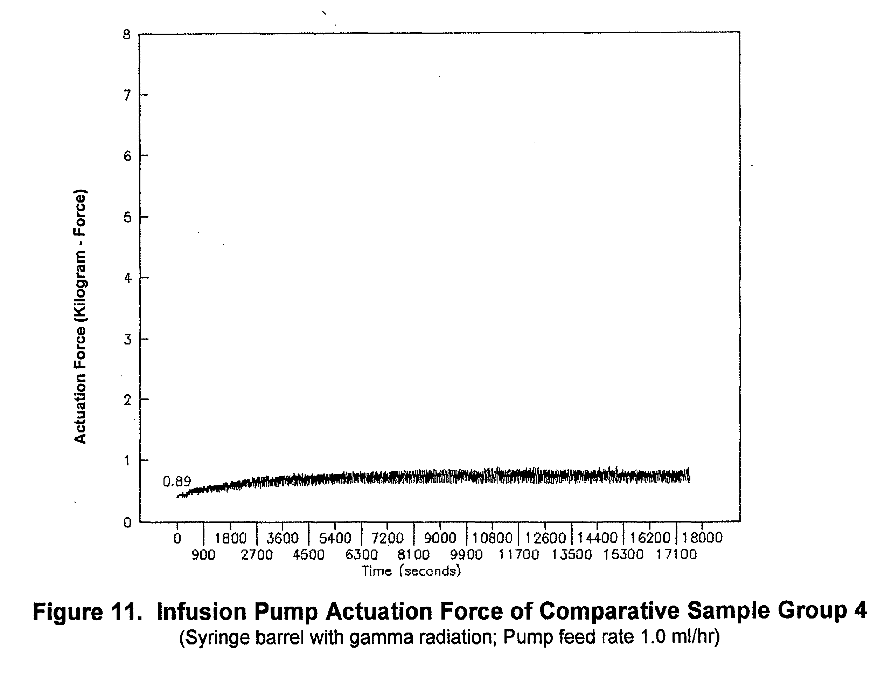

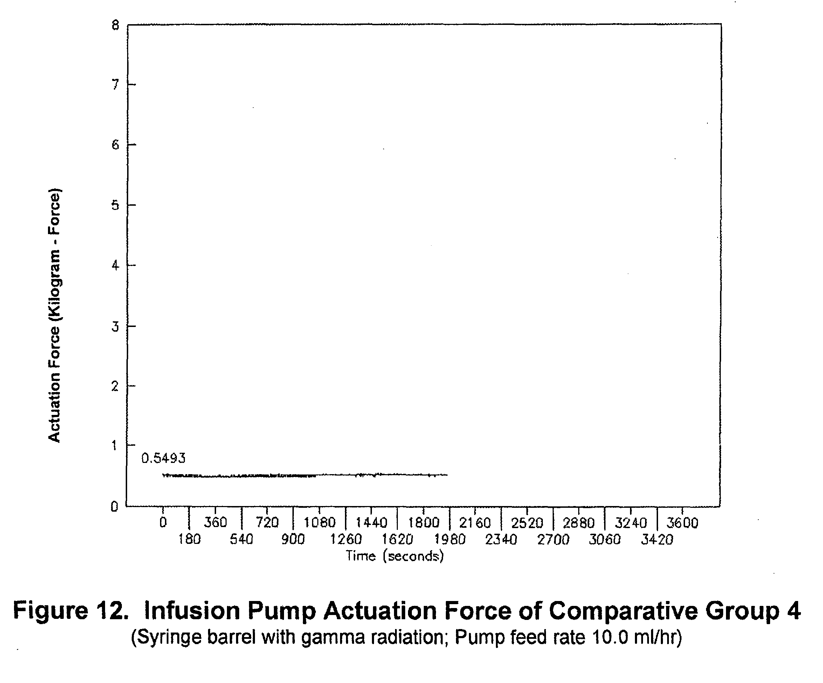

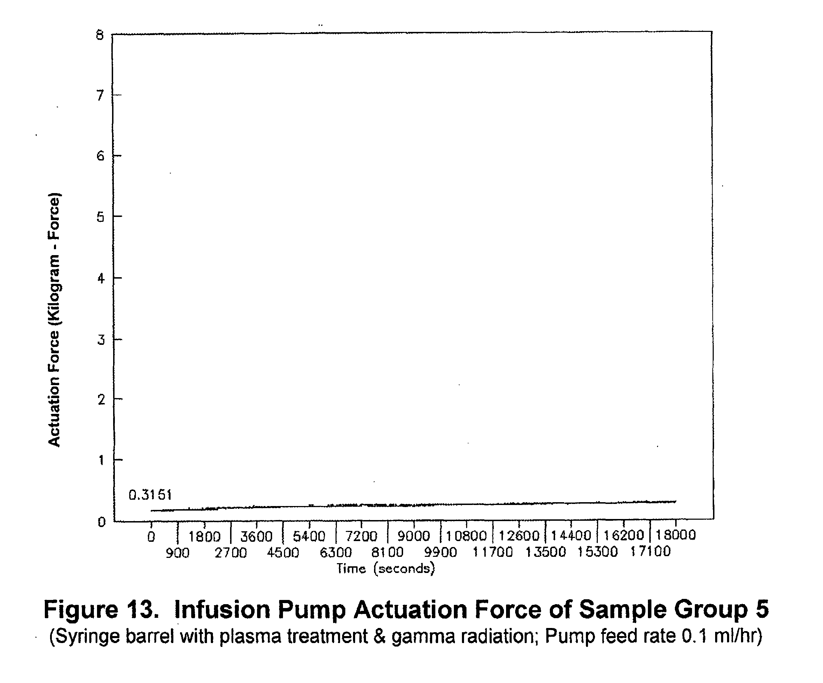

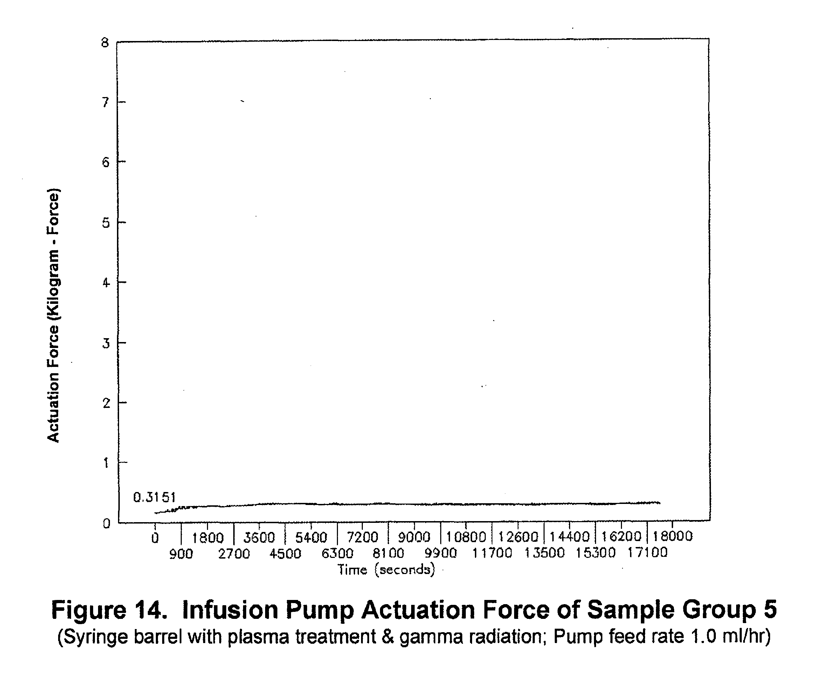

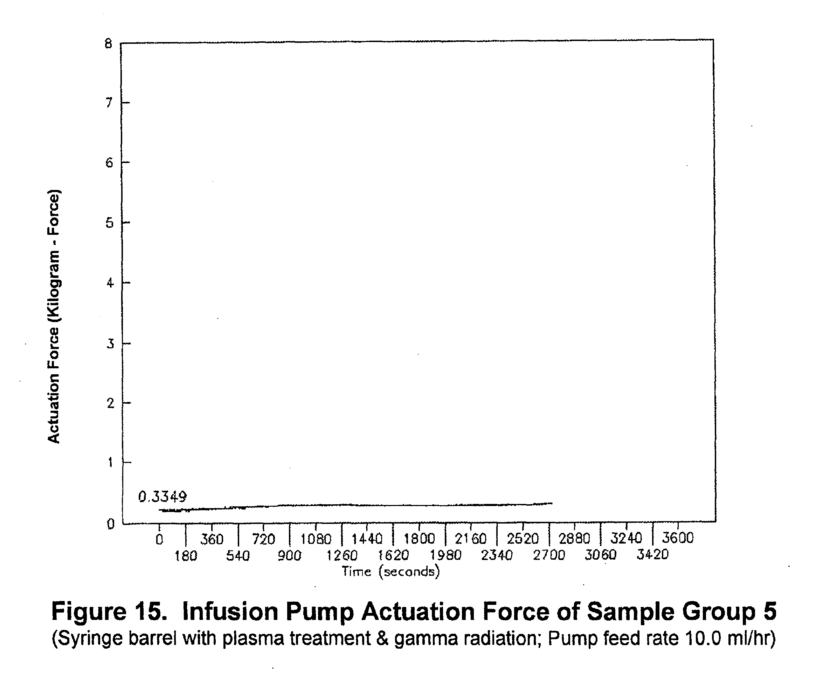

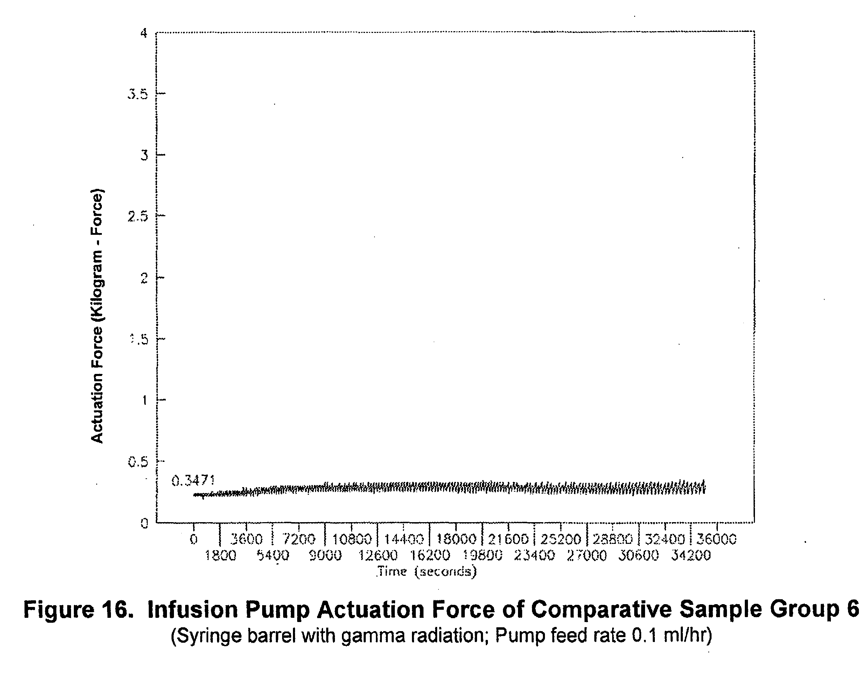

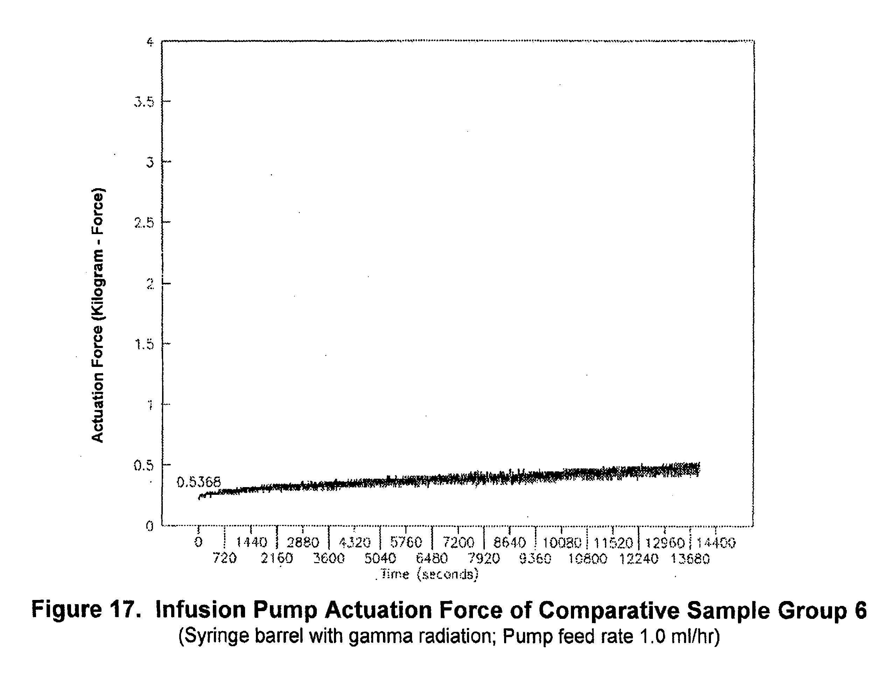

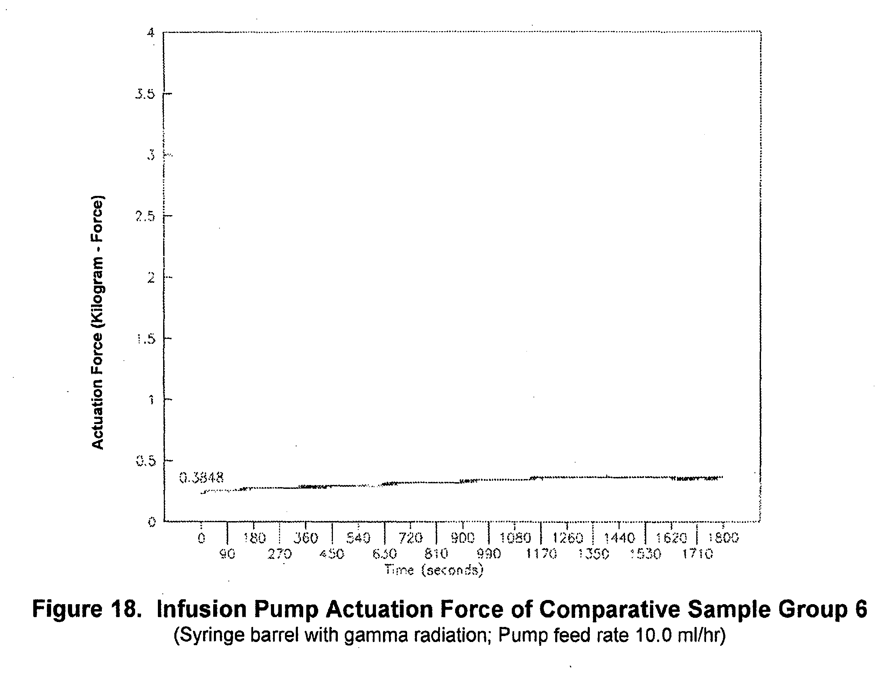

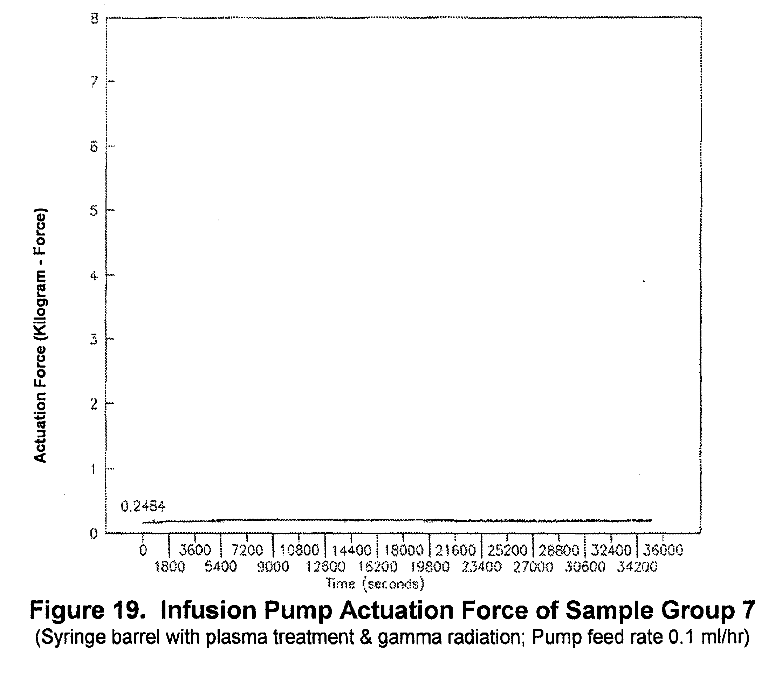

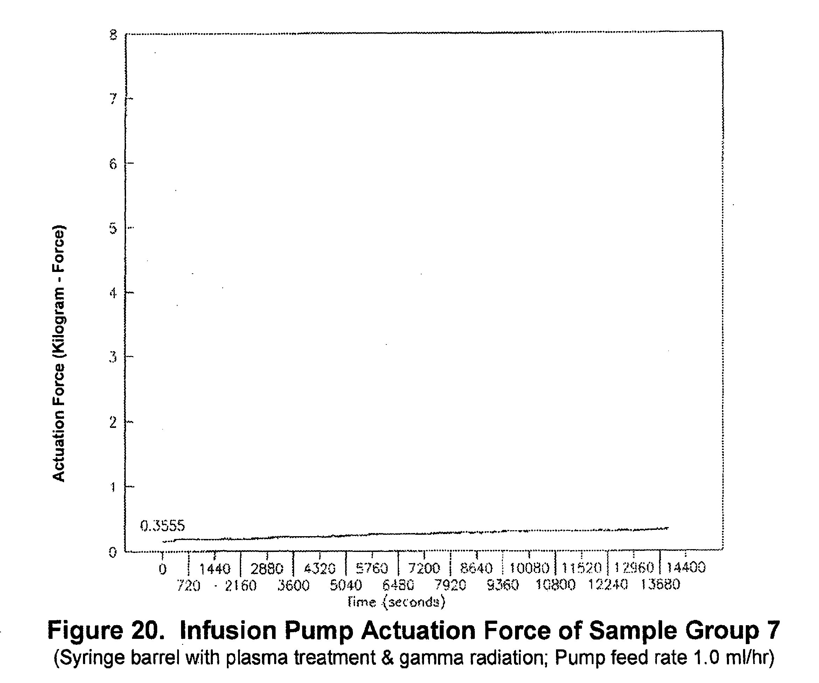

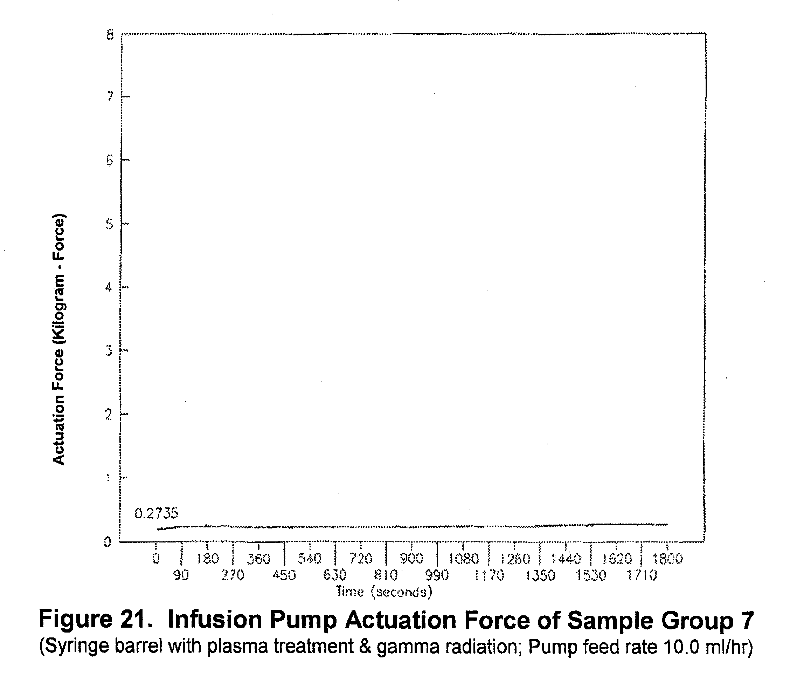

The syringe assemblies were evaluated for infusion pump actuation force according to ISO 7886-2 Annex A. A Becton Dickinson Program 2 syringe pump was used for testing at flow rates of 0.1 ml/hr, 1.0 ml/hr or 10.0 ml/hr. Force was measured using a force transducer placed between the syringe plunger rod and the displacement arm of the pump. A chart of force over time for each syringe was generated, as shown in Figs. 1-21. A visual determination of sticktion or no sticktion was made by viewing each chart for the smoothness of the curve. A smooth curve indicated no sticktion and an irregularly-shaped curve (for example with discernable peaks) indicated sticktion. The actuation force values reported in Table 1 below are the results of four samples for each of Sample Groups 1-12.

As shown in Table 1 below and with reference to Figs. 1-21, the 10 ml Sample Groups 3, 5, and 7 treated with both plasma treatment and radiation according to the present invention generally exhibited lower breakloose force and reduced sticktion, compared to the Sample Groups 1, 2, 4, and 6, respectively, treated with radiation or plasma alone. The 50 ml samples gave similar results. Sample Groups 9, 11, and 12 treated with both plasma treatment and radiation according to the present invention generally exhibited lower breakloose force, lower sustaining force, and reduced sticktion, compared to the Sample Groups 8 and 10 treated with radiation alone.

| **Table 1. Syringe Sample** | **10 ml** | | | | | | | **50 ml** | | | | |
|---|---|---|---|---|---|---|---|---|---|---|---|---|
| | 1 | 2 | 3 | 4 | 5 | 6 | 7 | 8 | 9 | 10 | 11 | 12 |
| Stopper | | | | | | | | | | | | |
| Butyl-A | x | x | x | | | | | x | x | x | x | x |
| Butyl-B | | | | x | x | | | | | | | |
| SBR | | | | | | x | x | | | | | |
| Lube w/12.5K cst Silicone | | | | | | | | x | x | | | |
| Lube w/100K cst Silicone | x | x | x | x | x | x | x | | | x | x | x |
| Gamma Irradiation 15 - 20 KGy | x | | x | | | | | x | x | | | |
| Gamma Irradiation 25 - 30 KGy | | | | x | x | | | | | x | x | x |
| Gamma Irradiation 30 - 35 KGy | | | | | | x | x | | | | | |
| Cyclic Olefin Polymer Barrel | | | | | | | | | | | | |
| Lube w/12.5K cst Silicone | x | x | x | x | x | x | x | x | x | x | x | |
| Lube w/(4:1) 12.5K cst Silicone: 1K cst Slianol | | | | | | | | | | | | x |
| Argon Plasma from Injection End (sec) | | 6 | 4 | | 6 | | 6 | | 4 | | 4 | 4 |
| Gamma Irradiation 15 - 20 KGy | x | | x | | | | | x | x | | | |
| Gamma Irradiation 25 - 30 KGy | | | | x | x | | | | | x | x | x |
| Gamma Irradiation 30 - 35 KGy | | | | | | x | x | | | | | |
| Assembly | | | | | | | | | | | | |
| Fill w/DI water & autoclave at 124°C for 30 min | x | x | x | x | x | x | x | x | x | x | x | x |
| Sustaining Force, Kgf (Boldface wlSticktion) | | | | | | | | | | | | |
| At 0.1 ml/h | 0.59 | 0.48 | 0.31 | 0.67 | 0.36 | 0.28 | 0.35 | 1.73 | 1.30 | 0.70 | 0.58 | 0.76 |
| At 1.0 ml/h | 1.75 | 0.98 | 0.47 | 0.67 | 0.41 | 0.49 | 0.37 | 2.68 | 1.99 | 1.45 | 0.98 | 1.06 |
| At 10.0 ml/h | 0.21 | 0.56 | 0.20 | 0.37 | 0.35 | 0.44 | 0.38 | 3.03 | 2.45 | 0.67 | 0.63 | 0.65 |
| Breakloose Force, Kgf | | | | | | | | | | | | |
| At 100 mm/min | 4.46 | 3.75 | 2.33 | 3.75 | 2.19 | 1.72 | 1.32 | 9.40 | 7.55 | 7.70 | 5.90 | 6.70 |
| Sustaining Force, Kgf | | | | | | | | | | | | |
| At 100 mm/min | 0.37 | 0.30 | 0.43 | 0.40 | 0.55 | 0.45 | 0.61 | 0.70 | 0.95 | 1.20 | 1.55 | 0.75 |

The present invention has been described with reference to specific details of particular embodiments thereof. It is not intended that such details be regarded as limitations upon the scope of the invention except insofar as and to the extent that they are included in the accompanying claims.

## Claims

1. A chamber for a medical article, the chamber having an inner surface adapted to sealingly engage an outer surface of a sealing member for a medical article, wherein the inner surface of the chamber has a coating thereon prepared from a composition comprising an organopolysiloxane, the coating being adhered to the inner surface by crosslinking induced by (1) oxidative treatment; and (2) (a) irradiation with an isotope, electron beam or ultraviolet radiation or (b) heat treatment, wherein the chamber is formed from cyclic polyolefin.

2. The chamber according to claim 1, wherein the chamber is selected from the group consisting of a syringe barrel, drug cartridge container, needleless injector container, liquid dispensing device container and liquid metering device container.

3. The chamber according to claim 1, wherein the organopolysiloxane is represented by the following structural formula (I): 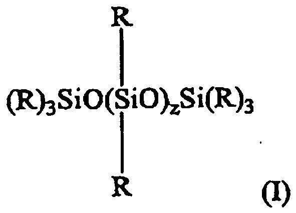 wherein R is alkyl and Z is 30 to 4,500.

4. The chamber according to claim 1, wherein the organopolysiloxane is polydimethylsiloxane.

5. The chamber according to claim 1, wherein the organopolysiloxane comprises at least two alkenyl groups, each alkenyl group being independently selected from the group consisting of vinyl, allyl, propenyl, butenyl, pentenyl, hexenyl, heptenyl, octenyl, nonenyl, and decenyl.

6. The chamber according to claim 1, wherein the organopolysiloxane comprises at least two polar groups, each polar group being independently selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, carboxypropyl, and fluoro groups.

7. The chamber according to claim 1, wherein the organopolysiloxane has a viscosity of 0.1 - 1000 Pa·s (100 to 1,000,000 mm²/s (centistokes)) preferably wherein the organopolysiloxane has a viscosity of 1 - 15 Pa·s (1,000 to 15,000 mm²/s (centistokes)) more preferably wherein the organopolysiloxane has a viscosity of 12.5 Pa·s (12,500 mm²/s (centistokes)).

8. The chamber according to claim 1, wherein the coated chamber is subjected to an oxidative treatment selected from the group consisting of atmospheric ionizing plasma treatment and vacuum ionizing plasma treatment.

9. The chamber according to claim 1, wherein the coated chamber is subjected to corona treatment.

10. The chamber according to claim 1, wherein the coated and oxidatively treated chamber is irradiated with gamma radiation, electron beam radiation or ultraviolet light, preferably wherein the coated and oxidatively treated chamber is irradiated with gamma radiation of 5 to 50 kiloGreys.

11. A medical article comprising:
(a) the chamber according to any of claims 1 to 10; and
(b) a sealing member having an exterior surface in sliding engagement with the interior surface of the chamber.

## Patentansprüche

1. Kammer für einen medizinischen Artikel, wobei die Kammer eine innere Fläche aufweist, die abdichtend an eine äußere Fläche eines Dichtungselements für einen medizinischen Artikel angreifen kann, wobei die innere Fläche der Kammer eine Beschichtung trägt, die aus einer Zusammensetzung hergestellt ist, welche ein Organopolysiloxan umfasst, wobei die Beschichtung durch Vernetzung an der inneren Fläche befestigt wird, welche durch (1) oxidative Behandlung und (2) (a) Bestrahlung mit einem Isotop, Elektronenstrahl oder ultravioletter Strahlung oder (b) Wärmebehandlung induziert wird, wobei die Kammer aus einem Cycloolefin-Polymer besteht.

2. Kammer gemäß Anspruch 1, wobei die Kammer aus der Gruppe ausgewählt ist, die aus einem Spritzenkolben, einem Medikamentenkartuschenbehälter, einem Behälter eines nadellosen Injektors, einem Behälter einer Flüssigkeitsabgabevorrichtung und einem Behälter einer Flüssigkeitsdosiervorrichtung besteht.

3. Kammer gemäß Anspruch 1, wobei das Organopolysiloxan durch die folgenden Strukturformel (I) dargestellt wird: 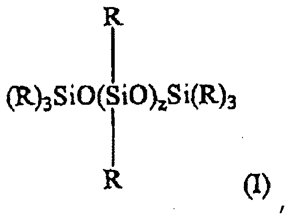 wobei R Alkyl ist und z = 30 bis 4500 ist.

4. Kammer gemäß Anspruch 1, wobei es sich bei dem Organopolysiloxan um Polydimethylsiloxan handelt.

5. Kammer gemäß Anspruch 1, wobei das Organopolysiloxan wenigstens zwei Alkenylgruppen umfasst, wobei jede Alkenylgruppe unabhängig aus der Gruppe ausgewählt ist, die aus Vinyl, Allyl, Propenyl, Butenyl, Pentenyl, Hexenyl, Heptenyl, Octenyl, Nonenyl und Decenyl besteht.

6. Kammer gemäß Anspruch 1, wobei das Organopolysiloxan wenigstens zwei polare Gruppen umfasst, wobei jede polare Gruppe unabhängig aus der Gruppe ausgewählt ist, die aus Acrylat-, Methacrylat-, Amino-, Imino-, Hydroxy-, Epoxy-, Ester-, Alkyloxy-, Isocyanat-, phenolischen, oligomeren Polyurethan-, oligomeren Polyamid-, oligomeren Polyester-, oligomeren Polyether-, Polyol-, Carboxypropyl- und Fluorgruppen besteht.

7. Kammer gemäß Anspruch 1, wobei das Organopolysiloxan eine Viskosität von 0,1 bis 1000 Pa.s (100 bis 1000 000 mm²/s (Centistokes)) aufweist, wobei das Organopolysiloxan vorzugsweise eine Viskosität von 1 bis 15 Pa.s (1000 bis 15000 mm²/s (Centistokes)) aufweist, wobei das Organopolysiloxan besonders bevorzugt eine Viskosität von 12,5 Pa.s (12500 mm²/s (Centistokes)) aufweist.

8. Kammer gemäß Anspruch 1, wobei die beschichtete Kammer einer oxidativen Behandlung unterzogen wurde, die aus der Gruppe ausgewählt ist, die aus einer ionisierenden Plasmabehandlung unter Atmosphärendruck und einer ionisierenden Plasmabehandlung unter Vakuum besteht.

9. Kammer gemäß Anspruch 1, wobei die beschichtete Kammer einer Koronabehandlung unterzogen wurde.

10. Kammer gemäß Anspruch 1, wobei die beschichtete und oxidativ behandelte Kammer mit Gammastrahlung, Elektronenstrahlung oder ultraviolettem Licht bestrahlt ist, wobei die beschichtete und oxidativ behandelte Kammer vorzugsweise mit Gammastrahlung von 5 bis 50 Kilogray bestrahlt ist.

11. Medizinischer Artikel, umfassend:
(a) die Kammer gemäß einem der Ansprüche 1 bis 10; und
(b) ein Dichtungselement, das eine äußere Fläche aufweist, die gleitend an der inneren Fläche der Kammer angreift.

## Revendications

1. Chambre pour un article médical, la chambre présentant une surface intérieure conçue pour coopérer de manière étanche avec une surface extérieure d'un élément d'étanchéité destiné à un article médical, dans laquelle la surface intérieure de la chambre est revêtue d'un revêtement préparé à partir d'une composition comprenant un polyorganosiloxane, le revêtement étant collé à la surface intérieure par réticulation induite par (1) traitement oxydant ; et (2) (a) irradiation avec un isotope, un faisceau électronique ou un rayonnement ultraviolet ou (b) traitement thermique, dans laquelle la chambre est formée à partir d'une polyoléfine cyclique.

2. Chambre selon la revendication 1, dans laquelle la chambre est choisie dans le groupe constitué d'un corps de seringue, d'un récipient de cartouche de médicament, d'un récipient d'injecteur sans aiguille, d'un récipient de dispositif distributeur de liquide et d'un récipient de dispositif doseur de liquide.

3. Chambre selon la revendication 1, dans laquelle le polyorganosiloxane est représenté par la formule structurale suivante (I) : 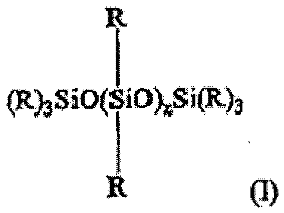 où R représente un alkyle et Z vaut de 30 à 4 500.

4. Chambre selon la revendication 1, dans laquelle le polyorganosiloxane est le polydiméthylsiloxane.

5. Chambre selon la revendication 1, dans laquelle le polyorganosiloxane comprend au moins deux groupements alcényle, chaque groupement alcényle étant choisi indépendamment dans le groupe constitué de vinyle, allyle, propényle, butényle, pentényle, hexényle, heptényle, octényle, nonényle et décényle.

6. Chambre selon la revendication 1, dans laquelle le polyorganosiloxane comprend au moins deux groupements polaires, chaque groupement polaire étant choisi indépendamment dans le groupe constitué de groupements acrylate, méthacrylate, amino, imino, hydroxy, époxy, ester, alkyloxy, isocyanate, phénolique, oligomère polyuréthane, oligomère polyamide, oligomère polyester, oligomère polyéther, polyol, carboxypropyle et fluoro.

7. Chambre selon la revendication 1, dans laquelle le polyorganosiloxane a une viscosité de 0,1 à 1 000 Pa·s (100 à 1 000 000 mm²/s (centistokes)), de préférence, dans laquelle le polyorganosiloxane a une viscosité de 1 à 15 Pa.s (1 000 à 15 000 mm²/s (centistokes)), de manière particulièrement préférée, dans laquelle le polyorganosiloxane a une viscosité de 12,5 Pa.s (12 500 mm²/s (centistokes).

8. Chambre selon la revendication 1, dans laquelle la chambre revêtue est soumise à un traitement oxydant choisi dans le groupe constitué du traitement au plasma ionisant atmosphérique et du traitement au plasma ionisant sous vide.

9. Chambre selon la revendication 1, dans laquelle la chambre revêtue est soumise à un traitement par effet corona.

10. Chambre selon la revendication 1, dans laquelle la chambre revêtue et soumise à un traitement oxydant est irradiée avec un rayonnement gamma, un rayonnement par faisceau électronique ou une lumière ultraviolette, de préférence, dans laquelle la chambre revêtue et soumise à un traitement oxydant est irradiée avec un rayonnement gamma de 5 à 50 kiloGreys.

11. Article médical comprenant :
(a) la chambre selon l'une quelconque des revendications 1 à 10 ; et
(b) un élément d'étanchéité présentant une surface extérieure en coopération coulissante avec la surface intérieure de la chambre.
